# EUROPÄISCHE PATENTSCHRIFT

(11) **EP 2 478 019 B1**
(45) Veröffentlichungstag und Bekanntmachung des Hinweises auf die Patenterteilung: **01.04.2015**
(21) Anmeldenummer: 10755128.5
(22) Anmeldetag: 13.09.2010
(51) Int. Cl.: C08F 220/00, C08F 226/06, C08F 290/06, C09D 4/00, C09D 171/00, C09D 201/00, A61K 9/00, A61K 47/00

(54) **MIT WIRKSTOFFHALTIGEN ÜBERZÜGEN BESCHICHTETE PELLETS**
PELLETS COATED WITH COATINGS CONTAINING ACTIVE SUBSTANCES
GRANULES RECOUVERTES D'ENROBAGES RENFERMANT DES SUBSTANCES ACTIVES

(30) Priorität: 17.09.2009 EP 09170553
(43) Veröffentlichungstag der Anmeldung: 25.07.2012
(73) Patentinhaber: BASF SE, 67056 Ludwigshafen (DE)
(72) Erfinder: KOLTER, Karl, 67117 Limburgerhof (DE); DJURIC, Dejan, 68165 Mannheim (DE); FISCHER, Stefan, 67251 Freinsheim (DE); KARL, Matthias, 68163 Mannheim (DE)
(86) Internationale Anmeldenummer: PCT/EP2010/063393
(87) Internationale Veröffentlichungsnummer: WO 2011/032916

(56) Entgegenhaltungen:
- WO-A1-2009/013202
- WO-A2-2007/051743

## Beschreibung

Die vorliegende Erfindung betrifft mit wirkstoffhaltigen Überzügen beschichtete Pellets, wobei der in Wasser schwerlösliche Wirkstoff in einem Überzug aus Copolymeren, die durch Polymerisation von Vinylacetat und N-Vinyllactamen in Gegenwart eines Polyethers erhalten werden, eingebettet vorliegt. Weiterhin betrifft die Erfindung Verfahren zur Herstellung solcher Pellets und deren Verwendung in pharmazeutischen Darreichungsformen.

Bei der Herstellung homogener Zubereitungen insbesondere von biologisch aktiven Substanzen hat die Solubilisierung von hydrophoben, also in Wasser schwerlöslichen Stoffen, eine sehr große praktische Bedeutung erlangt.

Unter Solubilisierung ist das Löslichmachen von in einem bestimmtem Lösungsmittel, insbesondere Wasser, schwer- oder unlöslichen Substanzen durch grenzflächenaktive Verbindungen, die Solubilisatoren, zu verstehen. Solche Solubilisatoren sind in der Lage, schlecht wasserlösliche oder wasserunlösliche Stoffe in klare, höchstens opaleszierende wässrige Lösungen zu überführen, ohne dass hierbei die chemische Struktur dieser Stoffe eine Veränderung erfährt.

Viele bekannte polymere Solubilisatoren weisen die Nachteile auf, dass sie keine stabilen festen Lösungen ausbilden. Außerdem lassen sie noch Raum für Verbesserungen, was die Solubilisierung in wässrigen Systemen betrifft. Auch hinsichtlich der Verarbeitbarkeit weisen einige der bekannten Solubilisatoren aufgrund ihrer Neigung zu Klebrigkeit Nachteile auf, da sie keine ausreichend fließfähigen Pulver darstellen.

Aus der DE-A 199 350 63 sind Polyalkylenoxid-haltige Pfropfpolymere auf Basis von Vinyllactamen und Vinylacetat sowie deren Verwendung als Gashydratinhibitoren bekannt.

Aus der WO 2007/051743 ist die Verwendung von wasserlöslichen oder wasserdispergierbaren Copolymerisatenaus N-Vinyllactam, Vinylacetat und Polyethern, als Solubilisatoren für pharmazeutische, kosmetische, lebensmitteltechnische, agrotechnische oder sonstige technische Anwendungen bekannt. Darin wird ganz allgemein beschrieben, dass die entsprechenden Pfropfpolymerisate auch in der Schmelze mit den Wirkstoffen verarbeitet werden können.

Aus der WO 2009/013202 ist bekannt, dass solche Pfropfpolymerisate aus N-Vinyllactam, Vinylacetat und Polyethern im Extruder aufgeschmolzen und mit pulverförmigen oder flüssigen Wirkstoffen vermischt werden können, wobei die Extrusion bei Temperaturen deutlich unter dem Schmelzpunkt des Wirkstoffs beschrieben ist.

Allerdings ist die Extrusion ein apparativ aufwendiges Verfahren. Außerdem kann es bei der Extrusion zu unerwünschten Temperaturbelastungen der Einsatzstoffe kommen.

Aufgabe der vorliegenden Erfindung war ein einfacheres Verfahren zur Einarbeitung schwerwasserlöslicher Substanzen in eine Formulierung mit verbesserter Löslichkeit, zu ermöglichen.

Demgemäß wurde ein Zubereitungen von in Wasser schwerlöslichen Wirkstoffen gefunden, die aus mit wirkstoffhaltigen Überzügen versehenen Trägerpartikeln bestehen, wobei die schwerlöslichen Wirkstoffe in Überzügen aus amphiphilen Copolymeren eingebettet vorliegen, und wobei die

amphiphilen Copolymere erhalten werden durch radikalisch initiierte Polymerisation einer Mischung aus
i) 40 bis 60 Gew.-% N-Vinyllactam
ii) 15 bis 35 Gew.-% Vinylacetat
iii) 10 bis 30 Gew.-% eines Polyethers
mit der Maßgabe, dass die Summe von i), ii) und iii) gleich 100 Gew.-% ist.

Weiterhin wurde ein Verfahren zur Herstellung solcher Zubereitungen gefunden, welches Verfahren dadurch gekennzeichnet ist, dass die Herstellung der Zubereitungen durch Versprühen einer Lösung, enthaltend einen oder mehrere Wirkstoffe und ein amphiphiles Copolymer, auf ein Wirbelbett aus Trägerpartikeln erfolgt.

Ganz besonders bevorzugt verwendete Polymerisate sind erhältlich aus
i) 50 bis 60 Gew.-% N-Vinyllactam
ii) 25 bis 35 Gew.-% Vinylacetat, und
iii) 10 bis 20 Gew.-% eines Polyethers,

Auch für die bevorzugten und besonders bevorzugten Zusammensetzungen gilt die Maßgabe, dass die Summe der Komponenten i), ii), und iii) gleich 100 Gew.-% beträgt.

Als N-Vinyllactam kommen N-Vinylcaprolactam oder N-Vinylpyrrolidon oder deren Mischungen in Betracht. Bevorzugt wird N-Vinylcaprolactam verwendet.

Als Pfropfgrundlage dienen Polyether. Als Polyether kommen vorzugsweise Polyalkylenglykole in Betracht. Die Polyalkylenglykole können Molekulargewichte von 1000 bis 100000 Da [Dalton], vorzugsweise 1500 bis 35000 Da, besonders bevorzugt 1500 bis 10000 Da, aufweisen. Die Molekulargewichte werden ausgehend von der gemäß DIN 53240 gemessenen OH-Zahl bestimmt.

Die Glasübergangstemperaturen liegen im Bereich von 40 bis 120 °C.

Als besonders bevorzugte Polyalkylenglykole kommen Polyethylenglykole in Betracht. Weiterhin eignen sich auch Polypropylenglykole, Polytetrahydrofurane oder Polybutylenglykole, die aus 2-Ethyloxiran oder 2,3-Dimethyloxiran erhalten werden.

Geeignete Polyether sind auch statistische oder blockartige Copolymere von aus Ethylenoxid, Propylenoxid und Butylenoxiden gewonnenen Polyalkylenglykolen wie beispielsweise Polyethylenglykol-Polypropylenglykol-Blockcopolymere. Die Blockcopolymere können vom AB- oder vom ABA-Typ sein.

Zu den bevorzugten Polyalkylenglykolen gehören auch solche, die an einer oder an beiden OH-Endgruppen alkyliert sind. Als Alkylreste kommen verzweigte oder unverzweigte C₁- bis C₂₂-Alkylreste in Betracht, bevorzugt C₁-C₁₈-Alkylreste, beispielsweise Methyl-, Ethyl-, n-Butyl-, Isobutyl-, Pentyl-, Hexyl-, Octyl-, Nonyl-, Decyl-, Dodecyl-, Tridecyl- oder Octadecyl-Reste.

Allgemeine Verfahren zur Herstellung der erfindungsgemäßen Copolymerisate sind an sich bekannt. Die Herstellung erfolgt durch freie radikalisch initiierte Polymerisation, bevorzugt in Lösung, in nichtwässrigen, organischen Lösungsmitteln oder in gemischt nichtwässrigen/wässrigen Lösungsmitteln. Geeignete Herstellverfahren sind beispielsweise in der WO 2007/051743 und der WO 2009/013202 beschrieben, auf deren Offenbarung hinsichtlich des Herstellungsverfahrens ausdrücklich Bezug genommen wird.

Das Verfahren zeichnet sich dadurch aus, dass in gängigen Wirbelschichtgeräten Trägerpartikel mit einem Überzug, enthaltend schwerlöslichem Wirkstoff. beladen werden. Dabei wird der Wirkstoff zusammen mit einem geeigneten Polymer in einem organischen Lösungsmittel aufgelöst und auf die Trägerpartikel gesprüht.

Als Trägerpartikel eignen sich sphärische oder zumindest annähernd spärische Partikel, sogenannte "Nonpareilles". Die Nonpareilles bestehen gemäß einer Ausführungsform der Erfindung rein, also zu 100 Gew.-%, aus pharmazeutischen Hilfsstoffen. Die Nonpareilles können aus üblichen pharmazeutischen Hilfsstoffen wie z.B. Saccharose, Carrageenan, Stärke oder mikrokristalliner Cellulose bestehen. Sie sind in unterschiedlicher Größe verfügbar (100 - 2000 µm).

Als Lösungsmittel können alle Lösungsmittel Verwendung finden, in denen die Wirkstoffe ausreichend löslich sind und die bei Normaldruck bis 160°C verdampfbar sind. Solche Lösungsmittel sind: z.B. Ethanol, Methanol, Isopropanol, Aceton, Essigester, Dichlormethan, Chloroform, Dimethylformamid, Methylethylketon oder Mischungen davon. Üblicherweise beträgt die Konzentration des erfindungsgemäß verwendeten Polymers in der Lösung 1 bis 40 Gew.-%.

Üblicherweise beträgt das Verhältnis von Wirkstoff zu amphiphilem Copolymer zwischen 1:99 und 80:20, vorzugsweise zwischen 10:90 und 60:40. So resultieren im finalen getrockneten Polymerüberzug der Pellets Wirkstoffkonzentrationen von 1 bis 80%.

Die Anwendung kann natürlich auch unter Schutzgas (Stickstoff) erfolgen. Der Einsatz der gängigen organischen Lösungsmittel ohne Schutzgas erfordert jedoch Wirbelschichtanlagen in Ex-Ausführung. Ferner lässt sich der Prozess sowohl mit üblichen Top - spray und Rotor-Geräten wie auch in Geräten mit Wurster Einsatz durchführen. Besonders geeignet ist die sogenannte Strahlwirbelschicht (Procelltechnologie) für die Applikation der beschriebenen Formulierungen.

Neben den Wirbelschichtgeräten können auch andere Gerätschaften eingesetzt werden, in denen die Pellets durch Drehung von Kesseln oder durch einströmende Luft in Bewegung versetzt werden wie z.B. Dragierkessel, Horizontaltrommelcoater, Kugelcoater, Innojet-Geräte.

Die Zulufttemperaturen liegen üblicherweise zwischen 30 und 200°C, vorzugsweise zwischen 40 und 120°C. Die Produkttemperaturen betragen in der Regel zwischen 25 und 100, vorzugsweise zwischen 30 und 80°C.

Gemäß einer bevorzugten Ausführungsform werden keine Nonpareilles als Trägerpartikel verwendet, sondern Partikel mit der gleichen oder zumindest ähnlichen Zusammensetzung wie der Überzug, nämlich mit Wirkstoff und amphiphilem Copolymer. Diese Starterpartikel können mit einem anderen Verfahren hergestellt worden sein, z.B. durch Granulation oder Extrusion. Sie können aber auch mit dem gleichen Verfahren erzeugt worden sein. Wird diese Vorlage nur in geringer Menge am Anfang eingesetzt - quasi als Starterkorn, damit der Prozess gestartet werden kann und größere Teilchen vorhanden sind, auf die die Sprühlösung aufgesprüht werden kann - so entstehen im Laufe des Prozesses Teilchen, die fast nur noch aus fester Lösung bestehen.

Die Schichtdicke der wirkstoffhaltigen Überzüge von amphiphilem Polymer kann 5 bis 1000, vorzugsweise 10 bis 700 µm betragen.

Der erfindungsgemäßen Formulierung können natürlich weitere pharmazeutisch übliche Hilfsstoffe zugesetzt werden, wie z.B. weitere Solubilisatoren, Polymere, Farbstoffe, anorganische Trägerstoffe, Sprengmittel, Gelbildner, Retardierungsmittel. Durch die Einarbeitung von magensaftresistenten Polymeren oder von Retardierungspolymeren lässt sich die Freisetzung des Wirkstoffes gezielt steuern.

Durch den Zusatz von kristallisationsinhibierenden Substanzen wie beispielsweise Kollidon 30 lässt sich die Stabilität der festen Lösungen erhöhen.

Die nach dem erfindungsgemäßen Verfahren erhaltenen Zubereitungen lassen sich grundsätzlich auf allen Gebieten einsetzen, bei denen in Wasser nur schwerlösliche oder unlösliche Substanzen entweder in wässrigen Zubereitungen zum Einsatz kommen sollen oder ihre Wirkung in wässrigem Milieu entfalten sollen.

Der Begriff "in Wasser schwerlöslich" umfasst erfindungsgemäß auch praktisch unlösliche Substanzen und bedeutet, dass für eine Lösung der Substanz in Wasser bei 20 °C mindestens 30 bis 100 g Wasser pro g Substanz benötigt wird. Bei praktisch unlöslichen Substanzen werden mindestens 10.000 g Wasser pro g Substanz benötigt.

Im Sinne der vorliegenden Erfindung sind unter in Wasser schwerlöslichen Substanzen vorzugsweise biologisch aktive Substanzen wie pharmazeutische Wirkstoffe für Mensch und Tier, kosmetische oder agrochemische Wirkstoffe oder Nahrungsergänzungsmittel oder diätetische Wirkstoffe zu verstehen.

Weiterhin kommen als zu solubilisierende schwerlösliche Substanzen auch Farbstoffe wie anorganische oder organische Pigmente in Betracht.

Als biologisch aktive Substanzen kommen erfindungsgemäß grundsätzlich alle festen Wirkstoffe in Betracht, die einen Schmelzpunkt aufweisen, der unter dem Zersetzungspunkt unter Extrusionsbedingungen der Copolymere liegt. Die Copolymere können im Allgemeinen bei Temperaturen bis zu 260 °C extrudiert werden. Die Temperaturuntergrenze richtet sich nach der Zusammensetzung der zu extrudierenden Mischungen und den jeweils zu verarbeitenden schwerlöslichen Substanzen.

Die verwendeten pharmazeutischen Wirkstoffe sind in Wasser unlösliche bzw. wenig lösliche Substanzen. Gemäß DAB 9 (Deutsches Arzneimittelbuch) erfolgt die Einstufung der Löslichkeit pharmazeutischer Wirkstoffe wie folgt: wenig löslich (löslich in 30 bis 100 Teilen Lösungsmittel); schwer löslich (löslich in 100 bis 1000 Teilen Lösungsmittel); praktisch unlöslich (löslich in mehr als 10000 Teilen Lösungsmittel). Die Wirkstoffe können dabei aus jedem Indikationsbereich kommen.

Als Beispiele seien hier Benzodiazepine, Antihypertensiva, Vitamine, Cytostatika - insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, , Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel genannt.

Besonders bevorzugt sind von den oben genannten pharmazeutischen Zubereitungen solche, bei denen es sich um oral applizierbare Formulierungen handelt.

Der Gehalt an erfindungsgemäßem Solubilisator in der pharmazeutischen Zubereitung liegt, abhängig vom Wirkstoff, im Bereich von 20 bis 99 Gew.-%.

Eine weitere besonders bevorzugte Ausführungsform bezieht sich auf pharmazeutische Zubereitungen, in denen die Wirkstoffe und das Copolymer als feste Lösung vorliegen. Dabei können die Entfernung des Lösungsmittels und die Einarbeitung der aktiven Substanz in einem Verfahrensschritt erfolgen. Hierbei beträgt das Gewichtsverhältnis von Copolymer zu Wirkstoff vorzugsweise von 1:1 bis 4:1, kann jedoch bis 100:1, insbesondere bis 15:1 betragen. Es kommt nur darauf an, dass bei Einsatz in der fertigen Arzneiform zum einen eine wirksame Menge Wirkstoff in der Arzneiform enthalten ist, und zum anderen bei oralen Arzneiformen die Formen nicht zu groß werden.

Zur Herstellung von pharmazeutischen Darreichungsformen wie beispielsweise Tabletten können die erhaltenen Zubereitungen mit üblichen pharmazeutischen Hilfsstoffen versetzt werden.

Dabei handelt es sich um Stoffe aus der Klasse der Füllstoffe, Weichmacher, Löslichkeitsvermittler, Bindemittel, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe, Weichmacher und Löslichkeitsvermittler.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Als Weichmacher eignen sich beispielsweise Triacetin, Triethylcitrat, Glycerolmonostearat, niedermolekulare Polyethylenglykole oder Poloxamere.

Als zusätzliche Löslichkeitsvermittler eignen sich grenzflächenaktive Substanzen mit einem HLB-Wert (HydrophilicLipophilicBalance) größer 11, beispielsweise mit 40 Ethylenoxid-Einheitten ethoxiliertes hydriertes Ricinusöl (Cremophor® RH 40), mit 35 Ethylenoxid-Einheiten ethoxiliertes Ricinusöl (Cremophor EL), Polysorbat 80, Poloxamere oder Natriumlaurylsulfat.

Als Schmiermittel können Stearate von Aluminium, Calcium, Magnesium und Zinn, sowie Magnesiumsilikat, Silikone und ähnliche verwendet werden.

Als Fließmittel können beispielsweise Talk oder kolloidales Siliciumdioxid eingesetzt werden.

Als Bindemittel eignet sich zum Beispiel mikrokristalline Cellulose.

Als Sprengmittel können quervernetztes Polyvinylpyrrolidon oder quervernetzte Natriumcarboxymethylstärke sein. Stabilisatoren können sein Ascorbinsäure oder Tocopherol.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandioxid oder Talkum, um die Lichtdurchlässigkeit zu erhöhen und um Farbstoffe einzusparen.

Die Zubereitungen können aber auch in anderen Dosierungsformen wie als Kapselfüllungen oder in Sachets eingesetzt werden.

Neben der Anwendung in der Kosmetik und Pharmazie eignen sich die erfindungsgemäß hergestellten Zubereitungen auch für den Einsatz im Lebensmittelbereich, zum Beispiel für die Einarbeitung von schwer wasserlöslichen oder wasserunlöslichen Nähr-, Hilfs- oder Zusatzstoffen, wie z.B. fettlösliche Vitamine oder Carotinoide. Als Beispiele seien mit Carotinoiden gefärbte Getränke genannt.

Die Anwendung der erfindungsgemäß erhaltenen Zubereitungen in der Agrochemie kann u. a. Formulierungen umfassen, die Pestizide, Herbizide, Fungizide oder Insektizide enthalten, vor allem auch solche Zubereitungen von Pflanzenschutzmitteln, die als Spritz- oder Gießbrühen zum Einsatz kommen.

Mit Hilfe des erfindungsgemäßen Verfahrens können wirkstoffhaltige Überzüge auf Nonpareilles als sogenannte feste Lösungen mit schwerlöslichen Substanzen erhalten werden. Als feste Lösungen werden erfindungsgemäß Systeme bezeichnet, in denen keine kristallinen Anteile der schwerlöslichen Substanz zu beobachten sind.

Bei visueller Begutachtung der stabilen festen Lösungen sind keine amorphen Bestandteile zu erkennen. Die visuelle Begutachtung kann mit einem Lichtmikroskop sowohl mit als auch ohne Polarisationsfilter bei 40facher Vergrößerung erfolgen. Weiterhin können die Zubereitungen auch mit Hilfe XRD (X-Ray Diffraction; Röntgendiffraktometrie) und DSC (Differential Scanning Calorimetry) auf Kristallinität oder Amorphizität untersucht werden.

Die nach dem erfindungsgemäßen Verfahren erhaltenen Zubereitungen liegen wie gesagt amorph vor, was bedeutet, dass die kristallinen Anteile der biologisch aktiven Substanz kleiner 5 Gew.-% betragen. Vorzugsweise wird der amorphe Zustand mittels DSC oder XRD überprüft. Solch ein amorpher Zustand kann auch als röntgenamorpher Zustand bezeichnet werden.

Das erfindungsgemäße Verfahren erlaubt die Herstellung von stabilen Zubereitungen mit hoher Wirkstoffbeladung und guter Stabilität bezüglich des amorphen Zustands der schwerlöslichen Substanz.

Die Besonderheit dieses Verfahrens liegt darin, dass ein amphiphiles Polymer zusammen mit schwerlöslichem Wirkstoff benutzt wird. Das amphiphile Polymer ist dabei in der Lage, nach Entfernung des Lösungsmittels den Wirkstoff in gelöster Form in der Überzugsschicht zu halten. Der Überzug ist damit kein üblicher Pellet- oder Tablettenüberzug, sondern ein Überzug, der schwerlöslichen Wirkstoff in gelöster Form hält. Der Vorteil fester Lösungen ist, dass schwerlösliche Wirkstoffe hiermit besser bioverfügbar gemacht werden können. Die Wirkstoffe können in der Überzugsschicht in amorpher oder molekular gelöster Form enthalten sein.

Ein weiterer Vorteil dieser Verarbeitungsmethode ist, dass feste Lösungen von schwerlöslichen Wirkstoffen zu multipartikulären festen Arzneiformen verarbeitet werden können. Diese multipartikulären Arzneiformen können beispielsweise in Hartgelatinekapseln gefüllt oder gar zu Tabletten verpresst werden.

Überraschenderweise weist das erfindungsgemäß als amphiphiles Polymer eingesetzte Polymer ausgezeichnete Binde- und Filmbildeeigenschaften auf, die unbedingt für diese Anwendung vonnöten sind. Aufgrund der mäßigen Sprühtrocknungseigenschaften des erfindungsgemäß verwendeten Polymers war diese Anwendung, bei der ebenfalls eine Lösung zerstäubt und getrocknet werden muss und zudem die Teilchen nicht miteinander verkleben dürfen, völlig unerwartet.

### Beispiele

### Herstellung des Copolymers

In einer Rührapparatur wurde die Vorlage ohne die Teilmenge von Zulauf 2 unter einer N₂-Atmosphäre auf 77°C aufgeheizt. Wenn die Innentemperatur von 77°C erreicht war, wurde die Teilmenge von Zulauf 2 zugegeben und 15 min anpolymerisiert. Anschließend wurden Zulauf 1 in 5h und Zulauf 2 in 2 h zudosiert. Nachdem alle Zuläufe zudosiert waren, wurde das Reaktionsgemisch noch 3h nachpolymerisiert. Nach der Nachpolymerisation wurde die Lösung auf einen Feststoffgehalt von 50 Gew.-% eingestellt.

| | |
|---|---|
| Vorlage: | 25 g Ethylacetat |
| | 104,0 g PEG 6000, |
| | 1,0 g von Zulauf 2 |
| | |
| Zulauf 1: | 240 g Vinylacetat |
| | 456 g Vinylcaprolactam |
| | 240 g Ethylacetat |
| | |
| Zulauf 2: | 10,44 g tert-Butylperpivalat (75 gew.%-ig in Aliphatengemisch) |
| | 67,90 g Ethylacetat |

Anschließend wurde das Lösungsmittel durch ein Sprühverfahren entfernt und ein pulverförmiges Produkt erhalten. Der K-Wert betrug 36, gemessen 1 gew.-%ig in Ethanol in. Die Glasübergangstemperatur betrug 70°C.

Der Zweischneckenextruder, der für die Herstellung der in den nachfolgenden Beispielen beschriebenen Formulierungen eingesetzt wurde, wies einen Schneckendurchmesser von 16 mm und eine Länge von 40D auf. Der gesamte Extruder war aus 8 einzeln temperierbaren Zylinderblöcken aufgebaut. Die ersten zwei Zylinder wurden zwecks besseren Materialeinzugs bei 20°C bzw. bei 70°C temperiert. Ab dem dritten Zylinder wurde eine konstante Temperatur eingestellt,

Die hergestellten festen Lösungen wurden mittels XRD und DSC auf Kristallinität bzw. Amorphizität unter Verwendung folgender Geräte und Bedingungen untersucht:
XRD
   Messgerät: Diffraktometer D 8 Advance mit 9-fach Probenwechsler (Fa.Bruker/AXS)
   Messart: θ- θ Geometrie in Reflexion
   Winkelbereich 2 Theta: 2-80°
   Schrittweite: 0,02°
   Messzeit pro Winkelschritt: 4,8s
   Divergence Slit: Göbelspiegel mit 0,4 mm Steckblende
   Antiscattering Slit: Sollerspalt
   Detektor: Sol-X Detektor
   Temperatur: Raumtemperatur
   Generatoreinstellung: 40kV/50mA
DSC
   DSC Q 2000 der Fa. TA -Instruments
   Parameter:
      Einwaage ca. 8,5 mg
      Heizrate: 20K/min

In den nachstehenden Beispielen wurden Saccharose-Pellets mit Partikelgrößen von 710-850 µm (Siebfraktion) mit wirkstoffhaltigen Überzügen versehen.

### Beispiel 1:

| Zusammensetzung | Menge |
|---|---|
| Ethanol | 1480 g |
| Copolymer | 80 g |
| Carbamazepin | 40 g |
| Saccharose Pellets | 1000 g |

**Wirbelschichtgranulator Glatt GPCG 3.1:**

| Prozessparameter | Werte |
|---|---|
| Volumenstrom [m³/h] | 140 |
| Zulufttemperatur [°C] | 50 |
| Sprühluftdruck [bar] | 1,5 |

Die XRD Analyse zeigte keine kristallinen Wirkstoffanteile.

Die Freisetzung des Wirkstoffs aus 518mg Pellets wurde in einer USP Apparatur 2 in 700mL 0,1 normaler HCL durchgeführt. Nach 60 Minuten waren 90 % des Wirkstoffs freigesetzt.

### Beispiel 2:

| Zusammensetzung | Menge |
|---|---|
| Aceton | 1600 g |
| Copolymer | 80 g |
| Fenofibrat | 40 g |
| Saccharose Pellets | 1000 g |

**Wirbelschichtgranulator Glatt GPCG 3.1:**

| Prozessparameter | Werte |
|---|---|
| Volumenstrom [m³/h] | 150 |
| Zulufttemperatur [°C] | 40 |
| Sprühluftdruck [bar] | 1,5 |

Die XRD Analyse zeigte keine kristallinen Wirkstoffanteile.

Die Freisetzung des Wirkstoffs aus 505mg Pellets wurde in einer USP Apparatur 2 in 700mL 0,1 normaler HCL durchgeführt. Nach 60 Minuten waren 100 % des Wirkstoffs freigesetzt.

### Beispiel 3:

| Zusammensetzung | Menge |
|---|---|
| Methanol | 1485 g |
| Copolymer | 80 g |
| Piroxicam | 40 g |
| MCC Pellets | 1000 g |

**Wirbelschichtgranulator Glatt GPCG 3.1:**

| Prozessparameter | Werte |
|---|---|
| Volumenstrom [m³/h] | 160 |
| Zulufttemperatur [°C] | 65 |
| Sprühluftdruck [bar] | 1,5 |

Die XRD Analyse zeigte keine kristallinen Wirkstoffanteile.

Die Freisetzung des Wirkstoffs aus 500mg Pellets wurde in einer USP Apparatur 2 in 700mL 0,1 normaler HCL durchgeführt. Nach 60 Minuten waren 100 % des Wirkstoffs freigesetzt.

### Beispiel 4:

| Zusammensetzung | Menge |
|---|---|
| Aceton | 1800 g |
| Copolymer | 90 g |
| Griseofulvin | 50 g |
| Stärke Pellets | 1000 g |

**Wirbelschichtgranulator Glatt GPCG 3.1:**

| Prozessparameter | Werte |
|---|---|
| Volumenstrom [m³/h] | 160 |
| Zulufttemperatur [°C] | 70 |
| Sprühluftdruck [bar] | 1,5 |

Die XRD Analyse zeigte keine kristallinen Wirkstoffanteile.

Die Freisetzung des Wirkstoffs aus 600mg Pellets wurde in einer USP Apparatur 2 in 700mL 0,1 normaler HCL durchgeführt. Nach 30 Minuten waren 100 % des Wirkstoffs freigesetzt.

### Beispiel 5:

| Zusammensetzung | Menge |
|---|---|
| Methanol | 1700 g |
| Copolymer | 130 g |
| Danazol | 60 g |
| Saccharose Pellets | 1000 g |

**Wirbelschichtgranulator Glatt GPCG 3.1:**

| Prozessparameter | Werte |
|---|---|
| Volumenstrom [m³/h] | 160 |
| Zulufttemperatur [°C] | 70 |
| Sprühluftdruck [bar] | 1,5 |

Die XRD Analyse zeigte keine kristallinen Wirkstoffanteile.

Die Freisetzung des Wirkstoffs aus 600 mg Pellets wurde in einer USP Apparatur 2 in 700mL 0,1 normaler HCL durchgeführt. Nach 30 Minuten waren 100 % des Wirkstoffs freigesetzt.

### Beispiel 6:

| Zusammensetzung | Menge |
|---|---|
| Ethanol | 1600 g |
| Copolymer | 60 g |
| Carbamazepin | 60 g |
| Carrageenan Pellets | 1000 g |

**Wirbelschichtgranulator Glatt GPCG 3.1:**

| Prozessparameter | Werte |
|---|---|
| Volumenstrom [m³/h] | 140 |
| Zulufttemperatur [°C] | 50 |
| Sprühluftdruck [bar] | 1,5 |

Die XRD Analyse zeigte keine kristallinen Wirkstoffanteile.

Die Freisetzung des Wirkstoffs aus 500mg Pellets wurde in einer USP Apparatur 2 in 700mL 0,1 normaler HCL durchgeführt. Nach 120 Minuten waren 100 % des Wirkstoffs freigesetzt.

### Beispiel 7:

| Zusammensetzung | Menge |
|---|---|
| Methanol | 2800 g |
| Copolymer | 160 g |
| Ketokonazol | 80 g |
| Saccharose Pellets | 1000 g |

**Wirbelschichtgranulator Glatt GPCG 3.1:**

| Prozessparameter | Werte |
|---|---|
| Volumenstrom [m³/h] | 150 |
| Zulufttemperatur [°C] | 55 |
| Sprühluftdruck [bar] | 1,5 |

Die XRD Analyse zeigte keine kristallinen Wirkstoffanteile.

Die Freisetzung des Wirkstoffs aus 480 mg Pellets wurde in einer USP Apparatur 2 in 700mL 0,1 normaler HCL durchgeführt. Nach 60 Minuten waren 100 % des Wirkstoffs freigesetzt.

### Beispiel 8:

| Substanz | Menge |
|---|---|
| Dichlormethan/Ethanol | 1520 g |
| Copolymer | 80 g |
| Itrakonazol | 40 g |
| Saccharose Pellets | 1000 g |

**Wirbelschichtgranulator Glatt GPCG 3.1:**

| Prozessparameter | Werte |
|---|---|
| Volumenstrom [m³/h] | 160 |
| Zulufttemperatur [°C] | 65 |
| Sprühluftdruck [bar] | 1,5 |

Die XRD Analyse zeigte keine kristallinen Wirkstoffanteile.

Die Freisetzung des Wirkstoffs aus 520 mg Pellets wurde in einer USP Apparatur 2 in 700mL 0,1 normaler HCL durchgeführt. Nach 80 Minuten waren 100 % des Wirkstoffs freigesetzt.

### Beispiel 9:

| Substanz | Menge |
|---|---|
| Methanol | 2000 g |
| Copolymer | 150 g |
| Piroxicam | 70 g |
| MCC Pellets | 1000 g |

**Wirbelschichtgranulator Glatt GPCG 3.1:**

| Prozessparameter | Werte |
|---|---|
| Volumenstrom [m³/h] | 160 |
| Zulufttemperatur [°C] | 65 |
| Sprühluftdruck [bar] | 1,5 |

Die XRD Analyse zeigte keine kristallinen Wirkstoffanteile.

Die Freisetzung des Wirkstoffs aus 500mg Pellets wurde in einer USP Apparatur 2 in 700mL 0,1 normaler HCL durchgeführt. Nach 60 Minuten waren 100 % des Wirkstoffs freigesetzt.

### Beispiel 10:

| Substanz | Menge |
|---|---|
| Aceton | 1000 g |
| Copolymer | 80 g |
| Fenofibrat | 20 g |
| Saccharose Pellets | 1000 g |

**Wirbelschichtgranulator Glatt GPCG 3.1:**

| Prozessparameter | Werte |
|---|---|
| Volumenstrom [m³/h] | 152 |
| Zulufttemperatur [°C] | 40 |
| Sprühluftdruck [bar] | 1,5 |

Die XRD Analyse zeigte keine kristallinen Wirkstoffanteile.

Die Freisetzung des Wirkstoffs aus 505mg Pellets wurde in einer USP Apparatur 2 in 700mL 0,1 normaler HCL durchgeführt. Nach 60 Minuten waren 100 % des Wirkstoffs freigesetzt.

### Beispiel 11:

| Substanz | Menge |
|---|---|
| Methanol | 3000 g |
| Copolymer | 200 g |
| Danazol | 85 g |
| MCC Pellets | 1000 g |

**Wirbelschichtgranulator Glatt GPCG 3.1:**

| Prozessparameter | Werte |
|---|---|
| Volumenstrom [m³/h] | 160 |
| Zulufttemperatur [°C] | 70 |
| Sprühluftdruck [bar] | 1,5 |

Die XRD Analyse zeigte keine kristallinen Wirkstoffanteile.

Die Freisetzung des Wirkstoffs aus 600mg Pellets wurde in einer USP Apparatur 2 in 700mL 0,1 normaler HCL durchgeführt. Nach 30 Minuten waren 100 % des Wirkstoffs freigesetzt.

### Beispiel 12:

Als Startkorn wurde gemahlene feste Lösung (Extrudat <500µm) aus Copolymer und Carbamazepin (Zusammensetzung 60:40) verwendet.

**Sprühlösung:**

| Substanz | Menge |
|---|---|
| Ethanol | 3000 g |
| Copolymer | 180 g |
| Carbamazepin | 100 g |

**Sprühgranulator ProCell 5:**

| Prozessparameter | Werte |
|---|---|
| Volumenstrom [m³/h] | 200 |
| Zulufttemperatur [°C] | 50 |
| Sprühluftdruck [bar] | 1,5 |

Die XRD Analyse zeigte keine kristallinen Wirkstoffanteile.

Die Freisetzung des Wirkstoffs aus 518mg Pellets wurde in einer USP Apparatur 2 in 700mL 0,1 normaler HCL durchgeführt. Nach 90 Minuten waren 100 % des Wirkstoffs freigesetzt.

### Beispiel 13:

Als Startkorn wurde gemahlene feste Lösung (Extrudat <500µm) aus Copolymer und Danazol (Zusammensetzung 70:30) verwendet.

**Sprühlösung:**

| Substanz | Menge |
|---|---|
| Methanol | 3000 g |
| Copolymer | 250 g |
| Danazol | 80 g |

**Sprühgranulator ProCell 5:**

| Prozessparameter | Werte |
|---|---|
| Volumenstrom [m³/h] | 220 |
| Zulufttemperatur [°C] | 60 |
| Sprühluftdruck [bar] | 1,5 |

Die XRD Analyse zeigte keine kristallinen Wirkstoffanteile.

Die Freisetzung des Wirkstoffs aus 518mg Pellets wurde in einer USP Apparatur 2 in 700mL 0,1 normaler HCL durchgeführt. Nach 45 Minuten waren 100 % des Wirkstoffs freigesetzt.

## Patentansprüche

1. Zubereitungen von in Wasser schwerlöslichen Wirkstoffen, bestehend aus mit wirkstoffhaltigen Überzügen versehenen Trägerpartikeln, wobei die schwerlöslichen Wirkstoffe in Überzügen aus amphiphilen Copolymeren eingebettet vorliegen, und wobei die amphiphilen Copolymere durch radikalisch initiierte Polymerisation einer Mischung aus:
i) 40 bis 60 Gew.-% N-Vinyllactam
ii) 15 bis 35 Gew.-% Vinylacetat
iii) 10 bis 30 Gew.-% eines Polyethers,
mit der Maßgabe, dass die Summe von i), ii) und iii) gleich 100 Gew.-% ist, erhalten werden.

2. Zubereitungen nach Anspruch 1, wobei die Copolymere, erhalten werden aus:
i) 50 bis 60 Gew.-% N-Vinyllactam
ii) 25 bis 35 Gew.-% Vinylacetat, und
iii) 10 bis 20 Gew.-% eines Polyethers.

3. Zubereitungen nach einem der Ansprüche 1 oder 2, wobei als Trägerpartikel Pellets aus pharmazeutischen Hilfsstoffen eingesetzt werden.

4. Zubereitungen nach einem der Ansprüche 1 bis 3, wobei die Trägerpartikel aus Saccharose, Carrageenan, Stärke oder mikrokristalliner Cellulose bestehen.

5. Zubereitungen nach einem der Ansprüche 1 bis 4, wobei die Trägerpartikel Partikelgrößen von 100 bis 2000 µm aufweisen.

6. Zubereitungen nach einem der Ansprüche 1 bis 3 und 5, wobei als Trägerpartikel Partikel, die aus dem Wirkstoff und amphiphilem Copolymer bestehen, eingesetzt werden.

7. Zubereitungen nach einem der Ansprüche 1 bis 6 , wobei die Überzüge zusätzlich pharmazeutische Hilfsstoffe enthalten.

8. Zubereitungen nach einem der Ansprüche 1 bis 7, wobei die Überzüge 20 bis 99 gew.-% an amphiphilen Copolymer enthalten.

9. Verfahren zur Herstellung von Zubereitungen gemäß einem der Ansprüche 1 bis 8 , **dadurch gekennzeichnet** ist, dass die Herstellung der Zubereitungen durch Versprühen einer Lösung, enthaltend einen oder mehrere Wirkstoffe und ein amphiphiles Copolymer in einem organischen Lösungsmittel, auf ein Wirbelbett aus Trägerpartikeln erfolgt.

10. Verfahren nach Anspruch 9 , **dadurch gekennzeichnet, dass** das organische Lösungsmittel bei Normaldruck bei Temperaturen kleiner 160 °C verdampfbar ist.

11. Verfahren nach Anspruch 9 oder 10 , **dadurch gekennzeichnet, dass** als Lösungmittel Ethanol, Methanol, Isopropanol, Aceton, Essigester, Dichlormethan, Chloroform, Dimethylformamid, Methylethylketon oder Mischungen davon eingesetzt werden.

12. Dosierungsformen, enthaltend Zubereitungen gemäß einem der Ansprüche 1 bis 8.

13. Dosierungsformen nach Anspruch 12 in Form von Tabletten, Kapseln oder Sachets.

## Claims

1. A formulation of sparingly water-soluble active ingredients, consisting of carrier particles provided with active ingredient-containing coatings, the sparingly soluble active ingredients being embedded in coatings composed of amphiphilic copolymers, and the amphiphilic copolymers being obtained by free-radically initiated polymerization of a mixture of
i) 40 to 60% by weight of N-vinyllactam
ii) 15 to 35% by weight of vinyl acetate
iii) 10 to 30% by weight of a polyether,
with the proviso that the sum of i), ii) and iii) is 100% by weight.

2. The formulation according to claim 1, wherein the copolymers are obtained from:
i) 50 to 60% by weight of N-vinyllactam
ii) 25 to 35% by weight of vinyl acetate and
iii) 10 to 20% by weight of a polyether.

3. The formulation according to either of claims 1 and 2, wherein the carrier particles used are pellets composed of pharmaceutical excipients.

4. The formulation according to any of claims 1 to 3, wherein the carrier particles consist of sucrose, carrageenan, starch or microcrystalline cellulose.

5. The formulation according to any of claims 1 to 4, wherein the carrier particles have particle sizes of 100 to 2000 µm.

6. The formulation according to any of claims 1 to 3 and 5, wherein the carrier particles used are particles which consist of the active ingredient and amphiphilic copolymer.

7. The formulation according to any of claims 1 to 6, wherein the coatings additionally comprise pharmaceutical excipients.

8. The formulation according to any of claims 1 to 7, wherein the coatings comprise 20 to 99% by weight of amphiphilic copolymer.

9. A process for producing formulations according to any of claims 1 to 8, which comprises producing the formulations by spraying a solution comprising one or more active ingredients and an amphiphilic copolymer in an organic solvent onto a fluidized bed composed of carrier particles.

10. The process according to claim 9, wherein the organic solvent is vaporizable at temperatures less than 160°C at standard pressure.

11. The process according to claim 9 or 10, wherein the solvent used is ethanol, methanol, isopropanol, acetone, ethyl acetate, dichloromethane, chloroform, dimethylformamide, methyl ethyl ketone or mixtures thereof.

12. A dosage form comprising formulations according to any of claims 1 to 8.

13. The dosage form according to claim 12 in the form of tablets, capsules or sachets.

## Revendications

1. Préparations de substances actives peu solubles dans l'eau, consistant en des particules de support munies d'enrobages contenant une substance active, les substances actives peu solubles étant incorporées dans des enrobages à base de copolymères amphiphiles, et les copolymères amphiphiles étant obtenus par polymérisation à amorçage radicalaire d'un mélange de :
i) 40 à 60 % en poids de N-vinyllactame
ii) 15 à 35 % en poids d'acétate de vinyle
iii) 10 à 30 % en poids d'un polyéther,
étant entendu que la somme de i), ii) et iii) est égale à 100 % en poids.

2. Préparations selon la revendication 1, dans laquelle les copolymères sont obtenus à partir de
i) 50 à 60 % en poids de N-vinyllactame
ii) 25 à 35 % en poids d'acétate de vinyle, et
iii) 10 à 20 % en poids d'un polyéther.

3. Préparations selon l'une quelconque des revendications 1 et 2, dans lesquelles on utilise comme particules de support des granules à base d'adjuvants pharmaceutiques.

4. Préparations selon l'une quelconque des revendications 1 à 3, dans lesquelles les particules de support sont constituées de saccharose, carraghénane, amidon ou cellulose microcristalline.

5. Préparations selon l'une quelconque des revendications 1 à 4, dans lesquelles les particules de support présentent des tailles de particules de 100 à 2 000 µm.

6. Préparations selon l'une quelconque des revendications 1 à 3 et 5, dans lesquelles on utilise comme particules de support des particules qui consistent en la substance active et un copolymère amphiphile.

7. Préparations selon l'une quelconque des revendications 1 à 6, dans lesquelles les enrobages contiennent en outre des adjuvants pharmaceutiques.

8. Préparations selon l'une quelconque des revendications 1 à 7, dans lesquelles les enrobages contiennent 20 à 99 % en poids de copolymère amphiphile.

9. Procédé pour la production de préparations selon l'une quelconque des revendications 1 à 8, **caractérisé en ce que** la production des préparations s'effectue par pulvérisation d'une solution contenant une ou plusieurs substances actives et un copolymère amphiphile dans un solvant organique, sur un lit tourbillonnaire de particules de support.

10. Procédé selon la revendication 9, **caractérisé en ce que** le solvant organique est évaporable sous la pression normale à des températures inférieures à 160 °C.

11. Procédé selon la revendication 9 ou 10, **caractérisé en ce qu'**on utilise comme solvant l'éthanol, le méthanol, l'isopropanol, l'acétone, l'acétate d'éthyle, le dichlorométhane, le chloroforme, le diméthylformamide, la méthyléthylcétone ou des mélanges de ceux-ci.

12. Formes galéniques, contenant des préparations selon l'une quelconque des revendications 1 à 8.

13. Formes galéniques selon la revendication 12, sous forme de comprimés, gélules ou sachets.
